## Europäisches Patentamt
## European Patent Office
## Office européen des brevets

(19)

(11) Veröffentlichungsnummer: **0 225 279**
**B1**

(12) # EUROPÄISCHE PATENTSCHRIFT

(45) Veröffentlichungstag der Patentschrift:
**20.12.89**

(21) Anmeldenummer: **86810486.0**

(22) Anmeldetag: **29.10.86**

(51) Int. Cl.⁴: **C03C 10/16,** C03C 10/08,
A61K 6/06, A61C 8/00,
A61C 5/08, A61C 13/083,
A61F 2/28

(54) Glimmer-Cordierit-Glaskeramik.

(30) Priorität: **04.11.85 DD 282404**
**04.11.85 DD 282400**
**04.11.85 DD 282401**
**04.11.85 DD 282402**
**04.11.85 DD 282403**

(43) Veröffentlichungstag der Anmeldung:
**10.06.87 Patentblatt 87/24**

(45) Bekanntmachung des Hinweises auf die Patenterteilung:
**20.12.89 Patentblatt 89/51**

(84) Benannte Vertragsstaaten:
**AT CH DE FR GB IT LI SE**

(56) Entgegenhaltungen:
**EP-A- 0 022 655**
**DD-A- 132 332**
**DD-A- 153 108**
**DE-A- 2 913 509**
**DE-A- 3 211 211**
**DE-A- 3 306 648**
**US-A- 3 997 352**
**US-A- 4 424 037**

(73) Patentinhaber: **VEB JENAer GLASWERK,**
**Otto-Schott-Strasse 13, DDR-6900 Jena(DD)**

(72) Erfinder: **Carl, Gunter, Dr. rer. nat.,**
**Wilhelm-Pieck-Strasse 26, DDR-6902 Jena-Lobeda(DD)**
Erfinder: **Naumann, Karin, Dr. rer. nat., Am**
**Planetarium 33, DDR-6900 Jena(DD)**
Erfinder: **Höland, Wolfram, Dr. rer. nat.,**
**Judith-Auer-Strasse 9, DDR-6902 Jena-Lobeda(DD)**
Erfinder: **Vogel, Werner, Prof. Dr. habil.,**
**Schlibachstrasse 11, DDR-6900 Jena(DD)**
Erfinder: **Beleites, Eggert, Dr., Nr. 41,**
**DDR-6901 Maua(DD)**
Erfinder: **Gudziol, Hilmar, Dr.,**
**Friedrich-Engels-Strasse 50, DDR-6900 Jena(DD)**
Erfinder: **Abert, Christine, Dr., Dessauer Strasse 39,**
**DDR-1143 Berlin(DD)**
Erfinder: **Knak, Günther, Prof. Dr.,**
**Otto-Winzer-Strasse 5, DDR-1142 Berlin(DD)**
Erfinder: **Hopp, Michael, Erich-Weinert-Strasse 16,**
**DDR-2337 Binz(DD)**
Erfinder: **Vogel, Frank, H. -Tops-Strasse 37,**
**DDR-1800 Brandenburg(DD)**
Erfinder: **Grosse, Steffen, Leninallee 60,**
**DDR-1017 Berlin(DD)**
Erfinder: **Jacobi, Ralf, Am Wiesengrund 22,**
**DDR-8909 Görlitz(DD)**
Erfinder: **Jungto, Harry, Dr. med. dent.,**
**Frauenhoferstrasse 3, DDR-6900 Jena(DD)**
Erfinder: **Musil, Rudolf, Prof. Dr. sc. med.,**
**Gillestrasse 14, DDR-6900 Jena(DD)**
Erfinder: **Kreisel, Lutz, Dr. med., Naumannstrasse 7,**
**DDR-6900 Jena(DD)**

(74) Vertreter: **Schweizer, Hans et al, Bovard AG**
**Patentanwälte VSP Optingenstrasse 16,**
**CH-3000 Bern 25(CH)**

**Beschreibung**

Die Erfindung betrifft eine Glimmer-Cordierit-Glaskeramik, die aufgrund ihrer Eigenschaften im weitesten Sinne im Maschinen- und Gerätebau oder auch als biokompatibler Werkstoff in der Medizin Einsatz findet. Sie findet vorzugsweise Anwendung für stomatologische Zwecke, wie beispielsweise für Zahnrestaurationen, insbesondere für Inlays, Kronen, Zahnaufbauten, Brücken und als Verblendschalen zur zahnfarbenen Verblendung von Kunststoffzähnen, Metallgerüsten oder natürlichen Zähnen, desweiteren in der Kieferorthopädie/Orthodontie, vorzugsweise für Klebebrackets und Klebeknöpfchen, als Fixationspunkte am Zahnschmelz für die Therapie mit festsitzenden Apparaturen und als Implantationsmaterial für die Kopf-, Hals-Chirurgie als bleibender Hartgewebeersatz in der Ohr-, Nasen-, Nasennebenhöhlen-, Schädelbasis- und Trachealchirurgie.

Sowohl glimmerhaltige Glaskeramiken oder Glaskeramiken, die als Hauptkristallphase Cordierit enthalten, sind bekannt.

Beispielsweise besitzen Cordierit-Glaskeramiken gemäß DE-OS 2.915.570 aufgrund des geringen linearen thermischen Ausdehnungskoeffizienten der Cordieritkristalle sehr gute thermische Eigenschaften, wie z. B. einen linearen thermischen Ausdehnungskoeffizient von 12 x 10$^{-7}$ K$^{-1}$. Gleichzeitig können in Cordierit-Glaskeramiken, wie z. B. nach US-PS 4.304.603 und DE-PS 3.130.977 solche vorteilhaften Spannungsbezirke im Glaskeramikmaterial erzeugt werden, daß ein Werkstoff mit beträchtlichen Festigkeiten von 250 - 300 MPa entwickelt werden kann, wobei Bruchzähigkeiten, d. h. K$_{IC}$-Werte bis 2,5 MPa m$^{1/2}$ erreicht werden.

Diese Glaskeramiken, die als Hauptkristallphase Cordierit enthalten sind jedoch nicht maschinell bearbeitbar, das heißt, sie können mit üblichen Hartmetallwerkzeugen nicht geformt werden.

Als Glaskeramikwerkstoffe mit vorteilhaften Bearbeitungseigenschaften sind Glimmerglaskeramiken, z. B. nach DE-AS 2.133.652, DE-OS 2.224.990 und DD-WP 113.885 bekannt.

Aufgrund der leichten Spaltbarkeit der ebenen Glimmerblättchen und der "Kartenhausanordnung" der Glimmerkristalle in der Glaskeramik, ist die gute Bearbeitbarkeit der Werkstoffe mit Hartmetallwerkzeugen möglich. Eine wesentliche Verbesserung der maschinellen Bearbeitbarkeit gegenüber den Glaskeramiken mit ebenen Glimmerblättchen, wie z. B. DE-AS 2.133.652, DE-OS 2.224.990, DD-WP 113.885 und solchen Glaskeramiken, die nahezu isolierte kugelförmige Anhäufungen von Fluorophlogopitkristallen aufweisen (wie in der US-PS 3.325.265 dargestellt) wurde durch die Entwicklung einer Glaskeramik erzielt, die einen neuen Typ gebogener Fluorophlogopitkristalle aufweist. Derartige, in der Patentschrift DD-WP 153.108 und nachfolgenden Publikationen, wie Glass Technology 24 (1983) 318, dargestellte Glaskeramiken zeigen ein Gefüge, in dem die 25 - 100 µm großen, gebogenen Fluerophlogopitkristalle in optimaler Konzentration in die Restglasmatrix eingebettet sind und sich die Kristalle berühren, so daß beste Voraussetzungen für eine optimale Bearbeitbarkeit geschaffen sind.

Obwohl für Glimmerglaskeramiken hinsichtlich der Bearbeitungseigenschaften mit der DD-WP 153.108 ein Optimum erzielt wurde, sind für eine breite Anwendung, besonders auch für medizinische Einsatzzwecke, von maschinell bearbeitbaren Glaskeramiken als Konstruktionswerkstoff besonders die Bruchzähigkeit, die Härte, die Druckfestigkeit, die Verschleißeigenschaften und der lineare thermische Ausdehnungskoeffizient der Werkstoffe noch nicht ausreichend.

Als Werkstoffe werden in der Stomatologie für Inlays, Kronen, Zahnaufbauten und Brücken vorzugsweise Edelmetalle und deren Legierungen, Sinterkeramiken und organische Polymere verwendet. Diese Werkstoffe besitzen jedoch erhebliche Mängel. Edelmetalle besitzen einen hohen Preis, weisen ein hohes thermisches Leitvermogen auf und besitzen eine ästhetisch nicht immer zufriedenstellende Farbe. Die Sinterkeramiken sind mit konventionellen Hartmetallwerkzeugen nicht bearbeitbar und besitzen durch den auftretenden Schrumpfungsvorgang Nachteile in der Verarbeitung. Die organischen Polymere besitzen vor allem eine ungenügende mechanische Festigkeit.

Auch die in der US-PS 4.431.420 und der EP-PS 22.655 sowie die in den US-PS 4.515.634 und der DE-OS 3.435.348 beschriebenen Glaskeramiken besitzen hinsichtlich der Anwendung in der Stomatologie wesentliche Nachteile, da deren Eigenschaften denen des Zahnschmelzes nicht optimal angepaßt sind. Das betrifft z. B. für die in der US-PS 4.431.420 und der EP-PS 22.655 beschriebene Tetrakieselsäureglimmer-Glaskeramik den linearen thermischen Ausdehnungskoeffizienten, der (nach "The Intern. J. of Periodontics und Restarat. Dent. 2(84) 36") 72 x 10$^{-7}$ K$^{-1}$ beträgt und damit beträchtlich unter dem Wert des Zahnschmelzes von 114 x 10$^{-7}$ K$^{-1}$ liegt. Solche beträchtlichen Unterschiede sind bereits bei geringsten Temperaturunterschieden Ursachen für unkontrollierte Spannungen zwischen Zahn und Glaskeramik, die zum Bruch des Materials und Randspaltbildung führen können. Weiterhin ist die Biegefestigkeit dieser Glaskeramik mit 50 - 55 MPa für eine breite umfangreiche Anwendung in der Stomatologie, besonders für dünnwandige Formkörper nicht ausreichend. Ein weiterer Nachteil der Tetrakieselsäureglimmerglaskeramik ist, daß offensichtlich aufgrund der Ausdehnungsproblematik, keine Vebundwerkstoffe mit hochfesten Grundkörpern, wie z. B. Korund oder Metalle, hergestellt werden können.

Von fluorophlogopithaltigen Glaskeramiken, wie sie z. B. in der DD-WP 113.885 und DD-WP 153.108 beschrieben werden, ist bekannt, daß sie sehr gute Bearbeitungseigenschaften aufweisen und solche Parameter besitzen, daß sie z. B. Anwendung im Geräte- und Maschinenbau finden. Ein Einsatz dieser Werkstoffe für stomatologische Zwecke würde jedoch aufgrund nicht optimal an den Zahnschmelz angepaßter Eigenschaften, z. B. thermische Eigenschaften, Härte, beträchtliche Nachteile mit sich bringen.

Als Materialien für Verblendschalen kommen neben Kunststoffen vorrangig Sinterkeramiken zur Anwendung.

Sinterkeramische Verblendschalen wurden industriell erstmalig nach Einführung der Kunststoffe Hekodent (1935), Paladon (1937/38) und Palapont (1940) in die Zahnheilkunde als sogenannte Schröder'sche Hohlzähne (Cavidens) gefertigt. Sie dienten als Außenschale für Kunstzähne aus den o. g. Kunststoffen zum Ausgleich der noch nicht ausreichenden Abriebfestigkeit.

Individuell gefertigte Verblendschalen aus Sinterkeramik sind ebenfalls zu dieser Zeit nachweisbar, wobei die Einführung der sogenannten Schalenkeramik durch Schröder 1932 diese Verblendtechnologie gefördert hat. Als Reaktion auf die unbefriedigenden Langzeitergebnisse der Kunststoffverblendung ist im Zeitraum 1952 - 1969 eine Bevorzugung sinterkeramischer Verblendschalen für Verblendkronen zu registrieren, wobei überwiegend auf sinterkeramische Fertigprodukte wie Goldknopf- und Platinlangstiftzähne zurückgegriffen wurde. Damals eingeführte Systeme mineralisch verblendeter Kronen basierten auf definierten Endformen von Schalenkörpern, die durch Ausschleifen von o. g. Vollkörperzähnen gewonnen wurden (Reumuth, E. und E. Arnold: Die Rostocker Facettenkrone. Dtsch. Stomat. 13 (1963) 391 - 398/Armbrecht, E. und A. Gerber: Die Schweriner Facettenkrone. Zahntechnik (Berlin) 5 (1964) 93 - 103). Mit der Entwicklung und Markteinführung der sogenannten Metallkeramik ist ein Rückgang der Schalentechnologie in den Ländern mit ausreichender Verfügbarkeit der Metallkeramik feststellbar, während bei Nichtverfügbarkeit eine industrielle Fertigung von sinterkeramischen Verblendschalen zur Erleichterung der Arbeit des Zahntechnikers induziert wurde (Richter, H.: Die Keracette, eine keramische Zahnschale. Zahntechnik (Berlin) 17 (1976) 313 - 316). Die Verfügbarkeit von Komposit-Kunststoffen führte zusammen mit der Einführung adhäsiver Techniken in Klinik und Zahntechnik zur Markteinführung sinterkeramischer Schalen neuen Typs, die zur "Fassadenrestaurierung" meist von Frontzähnen durch den Zahnarzt selbst genutzt wurden (Calamia. J.R.: Etched porcelain veneers: The current state of art. Quint. Int. 16 (1985) 5 - 12).

Sinterkeramische Verblendschalen lassen sich industriell nicht unter einer Mindeststärke von 1,5 bis 2 mm fertigen. Sie eignen sich von daher nicht für eine keramische Rindenschicht einer Verbundschale.

Von Hobo, S. und T. Iwata ist eine Hydroxyl-Apatit-Glaskeramik beschrieben wurden, die mittels Gußtechnologie zu Schalen zur Verblendung sichtbarer Zahnflächen geformt werden kann (Hobo, S. and T. Iwata, Castable apatite ceramics as a new biocompatible restorative material, I. Theoretical considerations, in Quit. Int. 16 (1985) 135 - 141). Diese Glaskeramik ist speziell für eine individuelle Fertigungstechnologie ausgelegt und erlaubt auch nur die Herstellung von Schalen mit größeren Wandstärken, ähnlich den Sinterkeramiken, und ist nicht geeignet für industrielle Fertigungstechnologien und die Hinterlegung von Kunststoff- oder Verbundschichten beispielsweise zur verbesserten Farbgestaltung. Ein Einsatz der bereits angeführten Glaskeramiken (US-PS 4.431.420, EP 22.655, US-PS 4.515.634, DE-OS 3.435.348, DD-WP 113.885 und DD-WP 153.108) für Verblendschalen ist nicht bekannt und die bereits aufgezeigten Mängel würden sich auch negativ bei einem solchen Einsatz auswirken.

Als Material für Klebebrackets wurde bisher Metall, Kunst stoff und Sinterkeramik beschrieben. Zur Zeit finden vorrangig Zahnregulierungselemente aus Metall, aufgrund ihrer guten mechanischen Stabilität und einer geringen Friktion des Regulierungsbogens im Schlitz, Anwendung. Die Ausgestaltung der Basisflächen der Zahnregulierungselemente aus Metall für orthodontische Adhäsive erfordert zur Erzielung ausreichender Verbundfestigkeiten aufwendige und komplizierte technologische Verfahren, wie z. B. nach DE-OS 2.618.952 die Erzeugung peripher perforierter Basen oder die Schaffung retentiver Netzbasen, fotogeätzter sphärischer Vertiefungen z. B. nach DE-OS 2.910.070 bzw. keilförmiger Rillfräsungen. Durch die Anwendung dieser aufwendigen Technologien sind bei Metallklebebrackets Verbundfestigkeiten bis maximal 12,3 MPa erreichbar (Diedrich, P. u. Dickmeiß, B. in Fortschr. Kieferorthop. 44 (1983), 298 - 310). Als ein Problem beim Einsatz von Metallklebebrackets hat sich erwiesen, daß verschiedentlich Korrosionserscheinungen am Bracket auftraten und eine permanente Verfärbung des Zahnschmelzes bewirkten, die nach Kappert u. a. (Fortschr. Kieferorthop. 45 (1984), 271 - 283) auf Crund und Fe-Spuren zurückzuführen ist. Die ästhetisch ungünstige Wirkung der Metallzahnregulierungselemente kann nach US-PS 4.527.975 durch eine zahnfarbene Kunststoffverblendung verbessert werden, wobei jedoch neben dem erheblichen materiellen und zeitlichen Aufwand für die Herstellung und Eingliederung dieser Verblendungen auch das Kariesrisiko für die Patienten wesentlich erhöht wird. Kunststoffbrackets, z. B. nach GB-PS 1.506.772, weisen gegenüber den Metallbrackets eine ästhetisch wesentlich günstigere Wirkung auf, die jedoch durch eine Verfärbung des Kunststoffs durch Nahrungs- und Genußmittel stark gemindert werden kann. Nachteilig bei Kunststoffbrackets ist auch deren geringe mechanische Festigkeit, die sich nach Dietrich (Fortschr. Kieferorthop. 42 (1981), 195 - 208) in einer mangelnden Torquefestigkeit, Slot-Deformation und Abrasion bzw. im Bruch der Bracketflügel zeigt, sowie das Auftreten hoher Reibung im Schlitz.

Auch durch den Einbau eines Metalleinsatzes in den Schlitz, wie z. B. nach DE 2.742.896, werden diese Nachteile nur unvollständig beseitigt.

Brackets aus Keramik, vorzugsweise aus $Al_2O_3$, wie sie in DE-OS 2.913.509 und US-PS 4.219.617 beschrieben werden, vereinigen eine ästhetisch günstige Wirkung und gute mechanische Festigkeit, und sind damit den Metall- bzw. Kunststoffbrackets überlegen. Als aufwendig und problematisch erweist sich jedoch bei den Keramikbrackets die Herstellung, da unter anderem die beim Sinterprozeß unkontrolliert auftretende Schrumpfung eine mechanische Nachbearbeitung unbendingt erforderlich macht, die aber nur mittels Diamantwerkzeugen zu realisieren ist. Gleichfalls ist eine individuelle Anpassung der Basisfläche der Keramikbrackets an die Zahnform, wie sie in bestimmten Fällen notwendig ist, nur sehr schwer möglich, da die Keramik aufgrund ihres Gefüges und der enthaltenen Kristallphasen nur sehr schlecht bearbeitbar ist. Nachteilig bei Keramikklebebrackets ist weiterhin, daß zur Erzielung ausreichender Verbundfestigkeiten Vertiefungen in der Basisfläche der Brackets zur besseren Adhäsion des orthodontischen Klebers erforderlich sind, die einen zusätzlichen Aufwand bei der Herstellung der Keramikklebebrackets bedingen.

Es ist bekannt, in der Kopf-Hals-Chirurgie $Al_2O_3$-Keramik oder Glaskeramik als Implantationsmaterial einzusetzen. Diese bekannten Glaskeramikmaterialien weisen aufgrund ihrer chemischen Stoffzusammensetzung ihrer ausgeschiedenen Kristallphasen und ihres Gefüges den Nachteil auf, daß sie schwer intraoperativ zu bearbeiten sind.

Weiterhin bekannt ist nach DE-OS 3.211.211 und DE-OS 3.211.209 eine Kombination von bioinertem und bioaktivem Material für Gehörknöchelchenprothesen. Der Nachteil dieser technischen Lösung besteht darin, daß sie aufgrund der Materialfestigkeit und ihrer bioinerten Oberflächenbeschichtung nicht intraoperativ bearbeitbar ist.

Außerdem muß ein großes Sortiment verschiedener, den anatomischen Gegebenheiten entsprechender Implantatkörper für den Operateur bereitstehen.

Ein weiterer Nachteil dieser bekannten Kombination unter Verwendung allgemein bekannter Glaskeramiken tritt dadurch auf, daß die Teilkomponenten unterschiedliche thermische Ausdehnungskoeffizienten haben, so daß insbesondere bei der Sterilisation Gefügestörungen an der Verbundstelle vorkommen können. Besonders bei großflächigen Implantatkörpern wirken sich die unterschiedlichen thermischen Ausdehnungskoeffizienten der Teilkomponenten nachteilig aus.

Das Ziel der Erfindung ist es, eine Glaskeramik zu entwickeln, die dem Werkstoff gegenüber dem bisher bekannten Stand der Technik hinaus Eigenschaften erreichen läßt, die völlig neue Einsatzgebiete erschließt, insbesondere für medizinische Zwecke, vorzugsweise in der Stomatologie und der Kopf-Hals-Chirurgie.

Aufgabe der Erfindung ist es, eine Glaskeramik zu entwickeln, die mit üblichen Hartmetallwerkzeugen ausgezeichnet bearbeitbar ist, gleichzeitig eine hohe Bruchzähigkeit und Härte sowie weitere gemäß der Anwendungszwecke einstellbare Eigenschaften aufweist, wobei die vorzugsweise Aufgabe der Erfindung weiterhin darin besteht, daß

1. hinsichtlich der Anwendung der Glaskeramik in der Stomatologie
- die Glaskeramik gut biologisch verträglich ist,
- die bisher vorhandenen Ausdehnungsunterschiede zwischen Zahn und künstlichem Dentalmaterial auf ein Minimum zu bringen sind,
- die zu geringe Härte und Festigkeit bisher benutzter Dentalmaterialien zu überwinden sind,
- ein Werkstoff einzusetzen ist, der eine ästhetisch bessere bzw. angepaßte Farbwirkung besitzt,
- die bisher eingesetzten sehr teuren Edelmetalle bzw. Legierungen durch Einsatz eines neuen Werkstoffes auf ökonomisch günstiger Rohstoffbasis zu ersetzen sind,
- die in der Glastechnik üblichen, einfachen Ur- und Umformungstechniken zu einem ökonomisch vorteilhaften Herstellungsprozeß anzuwenden sind;
2. hinsichtlich der Anwendung der Glaskeramik für Verblendschalen weiterhin
- eine einfache Anpassung und Ankopplung der Glaskeramik an einen Kunststoffzahn, ein Metallgerüst oder an einen natürlichen Zahn möglich ist,
- industrielle Fertigungsmethoden angewendet werden können;
3. hinsichtlich der Anwendung der Glaskeramik in der Kieferorthopädie/Orthodontie weiterhin
- die Glaskeramik eine hohe Retentionskapazität gegenüber othodontischen Klebern aufweist;
4. hinsichtlich der Anwendung der Glaskeramik in der Kopf-Hals-Chirurgie weiterhin
- die Glaskeramik sich intraoperativ mit den üblichen, während der Operation zur Verfügung stehenden Instrumenten gut bearbeiten läßt, so daß der Implantatkörper den individuell sehr unterschiedlichen anatomischen Gegebenheiten optimal angepaßt werden kann,
- eine Kombination der Glaskeramik mit einer hochbioaktiven Glaskeramik möglich ist.

Diese Aufgabe wird erfindungsgemäß dadurch gelöst, daß die Glaskeramik eine Zusammensetzung in Masse-% in den engen Grenzen von

| | |
|---|---|
| $SiO_2$ | 43–50 |
| $Al_2O_3$ | 26–30 |
| MgO | 11–15 |
| $R_2O$ | 7–10,5 |
| $F^-$ | 3,3–4,8 |
| $Cl^-$ | 0,01–0,6 |
| CaO | 0,1–3 |
| $P_2O_5$ | 0,1–5 |

besitzt, wobei $R_2O$ die Summe aus 3 - 5,5 Masse-% $Na_2O$ und 4 - 6 Masse-% $K_2O$ darstellt, und als Kristallphasen Glimmer neben 5 - 30 Vol.-% Cordierit enthält, wobei das Gefüge dadurch charakterisiert ist, daß relativ große, vorzugsweise gebogene, Glimmerkristalle von 10 - 200 μm Größe in die Glasmatrix eingebettet vorliegen und Cordieritkristalle von 0,5 - 5 μm Größe angeordnet sind, wobei auch kleine, ebene Glimmer von 0,5 - 5 μm Größe auftreten können.

Die Glimmer-Cordierit-Glaskeramik kann zur Verbesserung der physikalischen und chemischen Eigenschaften die Zusatzkomponenten BaO, SrO und PbO bis 8 Masse-% bzw. zur Farbgestaltung Farbkomponenten wie z. B. NiO, $Cr_2O_3$, $MnO_2$, FeO, $Fe_2O_3$ und $TiO_2$ bei 4 Masse-% einzeln oder im Gemisch enthalten.

Überraschend konnten die nachteiligen Eigenschaften von Glaskeramiken, die allein nur große gebogene Glimmerkristalle als Kristallphase enthalten, dadurch überwunden werden, daß durch eine gezielte doppelte Kristallisation des Ausgangsglases neben großen gebogenen Glimmerkristallen gleichzeitig kleine ebene Glimmerkristalle und/oder eine zweite Kristallphase, die Cordierit darstellt, zwischen den großen gebogenen Glimmerkristallen ausgeschieden werden.

Die Bildung der Cordieritkristallphase in der Glaskeramik konnte durch Auswertung von Röntgenbeugungsuntersuchungen und rasterelektronenmikroskopischen Untersuchungen eindeutig nachgewiesen werden.

Diese doppelte gesteuerte Kristallisation gelingt in dem an sich bekannten $SiO_2$-$Al_2O_3$-MgO-$Na_2O$-$K_2O$-$F^-$-System jedoch nur dann, wenn der erfindungsgemäße enge Zusammensetzungsbereich der Komponenten $SiO_2$, $Al_2O_3$, MgO, $Na_2O$, $K_2O$, $F^-$ gewählt wird und die Glaskeramik erfindungsgemäß gleichzeitig 0,01 - 0,6 Masse-% $Cl^-$, 0,1 - 3 Masse-% CaO und 0,1 - 5 Masse-% $P_2O_5$ enthält. Dieser erfindungsgemäße relativ enge Zusammensetzungsbereich ist eine Grundvoraussetzung dafür, daß die für eine gezielte doppelte gesteuerte Kristallisation notwendigen Mikrophasenbildungsprozesse im Ausgangsglas ablaufen können. Dabei ist charakteristisch, daß das Ausgangsglas aus einer tropfenförmigen Glasphase und einer glasigen Matrixphase besteht. Das wurde im elektronenmikroskopischen Bild eindeutig nachgewiesen. Die Tropfenglasphase stellt eine $Mg^{2+}$-, $Na^+$-, $K^+$-, $Al^{3+}$-reiche Silikatphase dar, die Glasmatrix ist $SiO_2$-reich. Aus der Tropfenglasphase bilden sich im Prozeß der thermischen Nachbehandlung die gebogenen Glimmerkristalle, die bekanntlich vor allem $Al^{3+}$-reicher sind als die ebenen Glimmerkristalle (Höland, Vogel et al. Glass Technology 24 (1983) 318). Mit fortschreitender Kristallisation tritt daher eine Verarmung an $Al^{3+}$- aber auch an $Mg^{2+}$-, $Na^+$-, $K^+$-, $F^-$-Ionen und (natürlich) $SiO_2$ im Restglas ein, so daß zunächst "nur noch" gerade blättchenförmige Glimmer und bei weiterer Verarmung der Glasphase vor allem an $Na^+$- und $K^+$-Ionen eine gezielte Cordieritkristallisation erfolgt. Daher sind die für die Kristallisation entscheidenden Diffusionsprozesse durch die unterschiedlichen Stadien der thermischen Behandlung genau steuerbar, so daß die Steuerung des Ablaufes der unterschiedlichen Kristallphasenbildung und die Festlegung des Kristallphasenanteils möglich ist.

Der Zusatz der Komponenten BaO, SrO und PbO bis 8 Masse-% sowie der Zusatz der Farbkomponenten, wie z. B. NiO, $Cr_2O_3$, $MnO_2$, FeO, $Fe_2O_3$, $TiO_2$ bis 4 Masse-% einzeln oder im Gemisch gibt dem Werkstoff neue zusätzliche gewünschte Färbungen, unterschiedliche Opazitätsgrade oder auch Fluoreszenz.

Die Herstellung des Ausgangsglases kann nach den in der Glastechnik bekannten Urformungsverfahren, wie z. B. Gießen, Pressen, Schleudern oder Ziehen zu einem Gegenstand erfolgen. Der Gegenstand wird anschließend auf Raumtemperatur abgekühlt oder direkt im Bereich von 600 - 1050°C in die erfindungsgemäße Glaskeramik überführt. Bei dieser in situ Kristallisation des Ausgangsglases ist es vorteilhaft, daß der Gegenstand in eine Form (z. B. Metall- oder Keramikmasse) eingebettet oder sogar unhüllt wird. Somit wird die Herstellung von Gegenständen mit sehr komplizierter äußerer Form möglich, ohne daß geringste Verformungen auftreten.

Die resultierende erfindungsgemäße Glaskeramik besitzt die besonderen Eigenschaftskombinationen von ausgezeichneter maschineller Bearbeitbarkeit, hoher Bruchzähigkeit bis 2,0 MPa $m^{1/2}$, einer Härte, als $HV_{0,07}$ bis 1000, einer Druckfestigkeit bis 450 $N/mm^2$, einem einstellbaren linearen thermischen Ausdehnungskoeffizienten von 75 - 125 $\times 10^{-7} K^{-1}$ und einer guten chemischen Beständigkeit sowie einer hohen Verschleißfestigkeit.

Dieser enorme Fortschritt in den Eigenschaftskombinationen, der gegenüber dem bisher bekannt gewordenen Stand der Technik erzielt wird, wird durch die gleichzeitige Ausscheidung von großen gebogenen Glimmerkristallen und kleinen ebenen Glimmerkristallen und/oder kleinen Cordieritkristallen in der erfindungsgemäßen Glaskeramik hervorgerufen. Die Ausbildung von Mikrospannungen durch die ausgeschiedenen Kristallphasen bewirkt dabei den entscheidenden Effekt für die gezielten Eigenschaftsvariationen und die sich daraus ergebenden neuen Einsatzgebiete, vorzugsweise in der Medizin.

Im Hinblick auf die Erzeugung spezieller Eigenschaften, wie z. B. hohe Festigkeit, einstellbaren Ausdehnungskoeffizienten, biologische Verträglichkeit u. a. wurden neben der Erzielung der ausgezeichneten Bearbeitungseigenschaften überraschend gefunden, daß Glimmer-Cordierit-Glaskeramiken für stomatologische Zwecke angewendet werden können. Dieser überraschende nützliche Effekt beruht darauf, daß in Glimmer-Cordierit-Glaskeramiken bessere mechanische, thermische und Verarbeitungseigenschaften bzw. in ihrer Gesamtheit neue physikalische Eigenschaften und chemische Eigenschaften erzeugt wurden als in bekannten Biomaterialien oder bekannten glimmerhaltigen Glaskeramiken.

Zunächst sind gute chemische Eigenschaften, z. B. hydrolytische Beständigkeit von glimmerhaltigen Glas keramiken bekannt. Überraschend konnte jedoch für die erfindungsgemäßen Produkte nachgewiesen werden, daß die Glaskeramiken im biologischen Milieu, beispielsweise in der Mundhöhle des Menschen, biokompatibel sind. Die überraschend nachgewiesenen neuen physikalischen Eigenschaften der Glimmer-Cordierit-Glaskeramiken sind z. B. der lineare thermische Ausdehnungskoeffizient, der bis 125 x $10^{-7}$ $K^{-1}$ variiert werden kann, die mechanische Festigkeit und Härte, die Abrasion der Materialien und die Verarbeitungseigenschaften des Ausgangsglases für die Herstellung der Glaskeramik bis hin zum Prozeß der Kermisierung und der Kombinationsmöglichkeit mit hochfesten Werkstoffen.

Erfindungsgemäß können Glimmer-Cordierit-Glaskeramiken in der Stomatologie vorzugsweise für Inlays, Kronen, Aufbauten und Brücken in beliebiger Form angewendet werden. Zur Aufrechterhaltung der optimalen mechanischen Parameter ist bei diesen Anwendungen jedoch eine Mindestwandstärke von 0,5 mm vorteilhaft.

Beispielsweise besitzt der Zahnschmelz (natürlicher Zahn) einen linearen thermischen Ausdehnungskoeffizienten von 114 x $10^{-7}$ $K^{-1}$. Die Glimmer-Cordierit-Glaskeramik besteht aus einem bisher nicht bekannten Gefüge, und zwar aus großen gebogenen und kleinen ebenen Fluorophlogopitkristallen und/oder Cordieritkristallen, die sich hochvernetzen. Daher besitzen diese Materialien einen Ausdehnungskoeffizienten, der direkt auf den des Zahnschmelzes eingestellt werden kann und ein Einsatz für Dentalrestaurationen, beispielsweise für Inlays, Kronen, Zahnaufbauten und Brücken erfindungsgemäß möglich ist. Damit ist ein wesentlicher Fortschritt gegenüber dem Stand der Technik erreicht worden, denn unkontrollierte Spannungen und Randspaltbildung zwischen Biomaterial und Zahn können vermieden werden, eine lange Funktionsperiode für Dentalprodukte im Seitenzahngebiet wird erreicht.

Die Variabilität bei der Einstellung des Ausdehnungskoeffizienten ist gleichzeitig eine Voraussetzung für Kombinationen der Glaskeramik mit hochfesten Werkstoffen, wie beispielsweise ein Verbundwerkstoff aus einer $Al_2O_3$- und Glimmer-Cordierit-Glaskeramik für einen Zahnaufbau - oder Brückenkonstruktion.

Von Glimmerglaskeramiken sind sehr gute Festigkeiten und Härteparameter bekannt. Für den Einsatz in der Stomatologie, wie vorzugsweise für Inlays, Kronen, Zahnaufbauten und Brücken, muß eine Vielzahl mechanischer Parameter optimale Werte im Vergleich zum Zahnschmelz einnehmen. So konnte überraschend gefunden werden, daß das Abrasionsverhalten von Glimmer-Cordierit-Glaskeramiken dem natürlichen Zahnschmelz ähnlich ist. Gleichzeitig besitzen die Materialien eine Härte von 300 - 800 (HV$_{0,07}$), Biegebruchfestigkeiten von mehr als 80 MPa (unterer Grenzwert), Druckfestigkeiten bis 450 N/mm$^2$ und eine Rauhtiefe von 0,15 µm und eine sehr gute chemische Beständigkeit, wie sie z. B. der Hydrolytischen Klasse 1 und 2 nach TGL 14809 bzw. RGW-Standard 1569 entspricht und die Materialien sind gleichzeitig ausgezeichnet maschinell bearbeitbar, so daß der Einsatz für die erfindungsgemäßen Glaskeramikdentalprodukte , wie durch die Ausführungsbeispiele dargestellt, möglich wurde. Außerdem wurde, gemessen an der Biegebruchfestigkeit, im Vergleich zur tetrakieselsäureglimmerhaltigen Glaskeramik der US-PS 4.431.420 ein wesentlicher Fortschritt erreicht, denn dieses Material brach bei einem unteren Grenzwert von ca. 50 MPa.

Im Vergleich zu Edelmetallen ist der erzielte Fortschritt der erfindungsgemäßen Anwendung der Cordierit-Glimmer-Glaskeramik für stomatologische Zwecke ohnehin offensichtlich. Zunächst wird z. B. durch das zahnähnliche Aussehen der Glaskeramik in bezug auf eine Krone aus Edelmetall bereits ein wesentlich verbesserter kosmetischer Effekt erreicht. Die hauptsächliche Wirkung wird jedoch auf ökonomischen Gebiet durch die wesentlich billigeren Rohstoffe erzielt. Gleichzeitig sind die Haupteigenschaften der Glaskermik denen des Zahnschmelzes wesentlich ähnlicher als die der Metalle (z. B. Wärmeleitfähigkeit, Ausdehnung, Abrasion, mechanische Eigenschaften). Besonders vorteilhaft ist auch, daß gegenüber bekannten Verfahren zur Herstellung von Sinterkeramiken Inlays, Kronen, Aufbauten, Brücken etc. aus Glimmer-Cordierit-Glaskeramik nach in der Glastechnik bekannten Ur- und Umformungsverfahren wie z. B. Pressen, Schleudern, Gießen hergestellt werden können.

Damit ist es möglich, je nach Anforderung, d. h. je nach anatomischer Besonderheit, gewünschte beliebige Formen von z. B. Inlays und Kronen oder Aufbauten und Brücken herzustellen. Im einzelnen besteht das Herstellungsverfahren darin, daß ein Ausgangsglas in Form von Glasfritte oder Kompaktmaterial bei 1450 - 1530°C erschmolzen wird und bei einer Temperatur von ca. 1250 - 1350°C, was einer Viskosität des Glases von $10^1$ bis $10^2$ Pas entspricht, in eine nach der gewünschten Zahnrestauration vorgefertigten und auf eine Temperatur von 500 - 1000°C vorgeheizten Muffel, gegossen wird.

Die Muffel besteht aus Einbettmassen, wie sie üblicherweise in zahntechnischen Labors, z. B. für den Modellguß von Prothesen aus Chrom-Cobalt-Molybdän- und Chrom-Nickel-Legierungen sowie Stahl verwendet worden. Der Gußkanal der Muffel sollte nicht länger als 10 mm sein und sein Durchmesser wenigstens 2 - 3 mm betragen. Zur besseren Entlüftung kann vorzugsweise ein Entlüftungskanal mit angebracht werden, was aber nicht generell erforderlich ist. Beim Gießen wird das vollständige Einbringen des Glases in die vorgefertigte Muffel zur Herstellung des jeweiligen Formkörpers durch ein Schleuderverfahren oder durch Anlegen von Vakuum erzielt.

Der Formkörper kann dann in der Muffel auf Raumtemperatur abgekühlt werden oder direkt im Temperaturbereich von 650°C bis 1050°C 0,5 bis 24 h in eine Glaskeramik überführt werden. In beiden Fällen ist eine ein- oder mehrstufige thermische Behandlung möglich. Wird die Muffel unmittelbar nach dem Urformungsprozeß auf Raum temperatur abgekühlt, so ist nach dem Ausbetten eine visuelle Einschätzung des Glaskörpers hinsichtlich eventuell auftretender Fehler im Urformungsprozeß (z. B. Risse, Sprünge, Blasen, nicht ausgeflossene Form) möglich und eine Aussonderung der fehlerhaften Glaskörper gegeben. Weist der Glaskörper keine Fehler auf, wird er mit 10 K/min auf eine Temperatur von 600 - 1000°C aufgeheizt und damit in die erfindungsgemäßen Glaskeramikdentalprodukte überführt. Diese thermische Behandlung wird im folgenden als Keramisierungsprozeß bezeichnet. Zur Gewährleistung der Formtreue ist es vorteilhaft, die Glaskörper vor dem Keramisieren wieder einzubetten. Nach dem Keramisierungsprozeß werden die Glaskeramikteile ausgebettet und durch Bearbeiten mit herkömmliche zahntechnischen Werkzeugen oder auch durch Abstrahlen mit feinkörnigem Sand von anhaftender Einbettmasse befreit. Durch Beschleifen und Polieren erreicht man optimal glatte und glänzende äußere Oberflächen, die eine Rauhtiefe bis zu 0,15 μm haben.

Nach den gleichen Verfahrensschritten, wie sie für die Herstellung von massiven Glimmer-Cordierit-Glaskeramiken für stomatologische Zwecke dargestellt wurden, können Verbundwerkstoffe aus einem hochfesten Grundkörper z. B. $Al_1O_3$-Sinterkeramik und der Glimmer-Cordierit-Glaskeramik hergestellt werden, wobei der Grundkörper völlig oder teilweise umhüllt ist. In diesem Fall wird der Grundkörper bereits in das Modell z. B. aus Wachs, eingebracht. Die entstandenen erfindungsgemäßen Dentalprodukte, z. B. Brücken oder Aufbauten, besitzen Festigkeiten von 200 - 400 MPa.

Ein weiterer positiver Effekt ergab sich dadurch, daß übliche Keramikmalfarben und/oder Glasuren auf die erfindungsgemäßen Glaskeramikdentalprodukte aufgebracht werden können. Dadurch wird z. B. eine individuelle Farbgebung möglich.

Eine ähnlich individuelle Farbgebung der Glimmer-Cordierit- Glaskeramiken wurde dadurch erzielt, daß die Zusatzkomponenten SrO, BaO, PbO bis 8 Masse-% und der Farbkomponenten FeO, $Fe_2O_3$, $MnO_2$, NiO, $Cr_2O_3$, $TiO_2$ bis 4 Masse-% eine Färbung bewirken,aber überraschenderweise den Phasenbildungsprozeß nicht wesentlich beeinflussen. Ein weiterer Vorteil der Anwendung von Glimmer-Cordierit-Glaskeramiken für stomatologische Zwecke besteht darin, daß aufgrund der ausgezeichneten maschinellen Bearbeitbarkeit die Herstellung durch Fräsen und/oder Bohren und/oder Drehen und/oder Sägen und/oder Schleifen erfolgen kann, ohne daß Diamantwerkzeuge erforderlich sind.

Erfindungsgemäß wird die Anwendung der Glimmer-Cordierit-Glaskeramik für Verblendschalen dadurch gelöst, daß die Verblendschale vestibulär aus einer Glaskeramikschicht und oral aus einer organischen Kunststoff- oder Verbundstoffschicht besteht, wobei beide Schichten mittels einer Haftvermittlerschicht verbunden sind, und wieterhin sowohl die günstigen Bearbeitungsmöglichkeiten sowie die guten physikalischen und chemischen Eigenschaften, die ästhetische Gestaltungsmöglichkeit und die Farbgebung der Glaskeramik optimal genutzt werden. Dadurch ist es möglich, Verblendschalen mit geringer Stärke zu fertigen, wobei die Glaskeramikschicht mittels in der Glastechnik bekannten Guß- oder Preßverfahren herstellbar ist. Damit ist eine industrielle Fertigungsmöglichkeit der genannten Verblendschale gegeben. Die individuelle Farbgebung der Verblendschale wird zusätzlich zur zahnfarbenen Glaskeramikschicht durch eine zahnfarbene organische Kunststoff- oder Verbundstoffschicht realisiert.

Die erfindungsgemäße vestibuläre Glaskeramikschicht gestattet eine Reduzierung der Schichtstärke auf eine Rindenschicht von gleich oder kleiner 0,5 mm, die ähnlich der Schmelzschicht des natürlichen Zahnes die Biokompatibilität der Verblendschale garantiert, die optischen Eigenschaften sichert und als Teil des Verbundkörpers die organische Kunststoff- oder Verbundstoffschicht gleich oder stärker 1 mm ausführen läßt. Damit kann die individuelle Anpassung einer industriemäßig gefertigten Verblendschale ausschließlich im Bereich der organischen Kunststoff- oder Verbundstoffschicht ohne Tangierung der Haftvermittlerschicht erfolgen. Gleichzeitig erlaubt die orale Hinterlegung der Glaskeramikschicht mit organischem Kunststoff oder Verbundstoff eine einfache Anpassung und Ankopplung an den zu verblendenden Körper. Die Aufbringung der Haftvermittlerschicht erfolgt in bekannter Weise durch den Einsatz siliziumorganischer Verbindungen, beispielsweise Tetraethoxysilan. Zur Verbesserung adhäsiver Eigenschaften trägt eine zur geometrischen Oberfläche relativ vergrößerte Oberflächenstruktur bei, die durch zweckmäßige Gestaltung der metallischen Preßform an der oralen Seite der vestibulären Glaskeramikschicht erzeugt und durch nachfolgende Konditionierungsverfahren verstärkt wird. Für eine weitere erfindungsgemäße Anwendung der Glimmer-Cordierit-Glaskeramik in der Kieferorthopädie/Orthodontie, vorzugsweise für Klebebrackets und Klebeknöpfchen, ist es von großer Bedeutung, daß bereits bei der Herstellung des Ausgangsglases durch Anwendung bekannter Urformungsverfahren, wie z. B. Gießen oder Pressen, eine weitgehende Vorformung der Klebebrackets und Klebeknöpfchen erfolgen kann.

Die Endfertigung der Teile kann aufgrund der ausgezeichneten maschinellen Bearbeitbarkeit der Glimmer-Cordierit-Glaskeramik durch Bearbeiten mit herkömmlichen Hartmetallwerkzeugen erfolgen, wobei dieser Prozeß einerseits durch die Herstellung standardisierter Grundkörper und andererseits automatisiert und damit ökonomisch vorteilhaft gestaltet werden kann.

Eine erforderliche individuelle Anpassung der Basisfläche der Klebebrackets an die Zahnform ist ebenfalls aufgrund der ausgezeichneten Bearbeitbarkeit der Glimmer-Cordierit-Glaskeramik z. B. durch Beschleifen mit in der zahnärztlichen Praxis gebräuchlichen Schleifkörpern sehr gut gewährleistet und bedeutet gegenüber den in DE-OS 2.913.509 und US-PS 4.219.617 beschriebenen Keramikbrackets einen wesentlichen Fortschritt.

Im Vergleich zu den Metallbrackets, wo aufwendige Verfahren zur Erzielung einer mikromechanischen Retentionsfläche für orthodontische Adhäsive erforderlich sind, bzw. im Vergleich zu Keramikbrackets, wo die Schaffung zusätzlicher Retentionshohlräume notwendig ist, wird bei den Glimmer-Cordierit-Glaskeramikklebebrackets bereits durch einen einfachen Feinschliff der Basisfläche eine ausgezeichnete mikromechanische Retentionsfläche erzielt, wodurch wesentlich höhere Verbundfestigkeitswerte als bei den Metall- bzw. Keramikklebebrackets erreicht werden. Weiterhin vorteilhaft für die Verbundfestigkeit zwischen Glaskeramikklebebrackets und Zahnschmelz ist die Möglichkeit, den linearen thermischen Ausdehnungskoeffizienten der Glimmer-Cordierit-Glaskeramik auf den Wert des Zahnschmelzes einstellen zu können und damit durch auftretende Temperaturschwankungen bedingte Spannungen zu vermeiden.

Daneben sind die ausgezeichneten physikalischen und chemischen Eigenschaften der Glimmer-Cordierit-Glaskeramik sowie deren gute biologische Verträglichkeit und Beständigkeit im Mundmilieu für die Anwendung in der Kieferorthopädie/Orthodontie von großer Bedeutung.

Das zahnfarbene Aussehen der Glimmer-Cordierit-Glaskeramik, das durch färbende Zusatzkomponenten noch verbessert werden kann, wirkt sich für den Patienten aus kosmetischer Sicht sehr günstig aus, wobei wesentlich ist, daß im Gegensatz zu den Kunststoffbrackets keine dauerhaften und nachteiligen Verfärbungen durch z. B. Nahrungs- und Genußmittel auftreten. Weiterhin zeichnen sich die erfindungsgemäßen Glaskeramikklebebrackets für den Patienten, aufgrund ihrer Formgestaltung und dadurch, daß die Kanten der Glaskeramikklebebrackets gebrochen bzw. abgerundet sind, durch angenehme Trageeigenschaften aus.

Erfindungsgemäß besteht das Implantatmaterial für die Hals-Kopf-Chirurgie alleinig aus der Glimmer-Cordierit- Glaskeramik oder aus einer Kombination einer maschinell bearbeitbaren, hochbioaktiven Glaskeramik mit der besagten biokompatiblen Glimmer-Cordierit-Glaskeramik. Die maschinell bearbeitbare, hochbioaktive Glaskeramik weist dabei eine Zusammensetzung in Masse-% wie folgt auf (DE-OS 3.306.648):

| | |
|---|---|
| $SiO_2$ | 19–52 |
| $Al_2O_3$ | 12–23 |
| $MgO$ | 5–15 |
| $Na_2O/K_2O$ | 3–10 |
| $CaO$ | 9–30 |
| $P_2O_5$ | 4–24 |
| $F$ | 0,5–7 |

Die Kombination der angegebenen biokompatiblen Glimmer-Cordierit-Glaskeramik mit der hochbioaktiven Glaskeramik erweist sich deshalb als günstig, weil sie in der Erweichungsphase aufgrund entsprechend abgestimmter linearer thermischer Ausdehnungskoeffizienten fest miteinander verbunden werden und dadurch bei Temperaturänderungen an der Phasengrenze keine Gefügestörungen eintreten können.

Für die Bearbeitung der Glaskeramikkombination ist die Kennzeichnung der Phasengrenze zwischen der hochbioaktiven Glaskeramik und der besagten Glimmer-Cordierit-Glaskeramik wichtig. Das geschieht dadurch, daß die Phasengrenze zwischen beiden zu verbindenden Glaskeramiken durch Einbringen geringer Farboxidmengen vor der thermischen Behandlung der Ausgangsprodukte als farbige Grenzschicht sichtbar gemacht wird.

Die erfindungsgemäße Verwendung der Glimmer-Cordierit-Glaskeramik hat den Vorteil einer geringen Löslichkeit, welche eine Langzeitformkonstanz garantiert. Außerdem besitzt wie eine hohe Oberflächenladung, die im Bereich der Ohren, der Nase und der Nasennebenhöhlen sowie der Trachea eine schnelle Sekundärepithelisierung bedingt. Insgesamt besitzt die erfindungsgemäße Verwendung der besagten Glaskeramik den Vorteil, daß sie intraoperativ mit den üblichen, während der Operation zur Verfügung stehenden Instrumenten zu bearbeiten ist.

Weiterhin ermöglicht die gute Blutverträglichkeit der erfindungsgemäßen Glimmer-Cordierit-Glaskeramik ihren Einsatz im kardiovaskulären System, beispielsweise für Herzpumpen.

Die Erfindung soll anhand von Ausführungsbeispielen näher erläutert werden.

Tabelle 1 zeigt einen Überblick über die chemische Zusammensetzung in Masse-% der erfindungsgemäßen Glaskeramiken bzw. deren Ausgangsgläser. In Tabelle 2 werden Beispiele gegeben, die den Zusammenhang zwischen chemischer Zusammensetzung, thermischer Behandlung der Ausgangsgläser, Kristallphasenzusammensetzung und Eigenschaften der erfindungsgemäßen Glaskeramiken aufzeigen. Es existieren eine Vielzahl von möglichen Eigenschaftskombinationen der erfindungsgemäßen Glaskeramiken, wie z. B. eine hohe Bruchzähigkeit mit einer guten bis sehr guten maschinellen Bearbeitbarkeit bei einem in weiten Grenzen einstellbaren linearen thermischen Ausdehnungskoeffizienten bzw. eine hohe Härte mit einer guten maschinellen Bearbeitbarkeit bei gleichzeitig sehr guter chemischer Beständigkeit.

Tabelle 1

Zusammensetzungen von Glimmer-Cordierit-Glaskeramiken bzw. deren Ausgangsgläsern

| | 1 | 2 | 3 | 4 | 5 | 6 | 7 | 8 | 9 | 10 | 11 |
|---|---|---|---|---|---|---|---|---|---|---|---|
| $SiO_2$ | 45,2 | 47,3 | 49,3 | 43,6 | 44,1 | 45,3 | 45,0 | 42,9 | 45,3 | 45,0 | 44,6 |
| $Al_2O_3$ | 29,6 | 25,7 | 26,4 | 26,3 | 27,6 | 28,1 | 25,9 | 26,0 | 26,3 | 28,5 | 26,9 |
| $MgO$ | 12,0 | 11,1 | 11,8 | 14,7 | 11,4 | 13,6 | 11,1 | 11,9 | 11,5 | 11,9 | 12,3 |
| $Na_2O$ | 3,9 | 3,1 | 3,3 | 5,2 | 4,2 | 4,3 | 3,3 | 3,0 | 3,2 | 3,7 | 3,5 |
| $K_2O$ | 4,5 | 4,8 | 4,0 | 4,3 | 5,8 | 4,8 | 5,1 | 3,8 | 4,6 | 4,3 | 4,6 |
| $F^-$ | 4,2 | 3,5 | 4,1 | 4,5 | 3,9 | 3,3 | 4,2 | 4,2 | 4,0 | 4,2 | 4,1 |
| $Cl^-$ | 0,1 | 0,05 | 0,5 | 0,2 | 0,1 | 0,4 | 0,1 | 0,05 | 0,3 | 0,05 | 0,1 |
| $CaO$ | 0,1 | 0,5 | 0,3 | 0,1 | 2,8 | 0,1 | 0,3 | 0,1 | 0,1 | 0,1 | 0,1 |
| $P_2O_5$ | 0,3 | 4,0 | 0,2 | 1,1 | 0,1 | 0,1 | 0,5 | 0,2 | 0,7 | 0,1 | 0,3 |
| $SrO$ | – | – | – | – | – | – | 4,5 | – | – | – | – |
| $PbO$ | – | – | – | – | – | – | – | 7,9 | – | – | – |
| $Fe_2O_3$ | – | – | – | – | – | – | – | – | 3,7 | – | – |
| $MnO_2$ | – | – | – | – | – | – | – | – | – | 2,2 | – |
| $TiO_2$ | – | – | – | – | – | – | – | – | 0,2 | – | 3,5 |

Tabelle 2

Thermische Behandlung der glasigen Produkte und Eigenschaften der resultierenden Glimmer-Cordierit-Glaskeramiken

| thermische Behandlung bzw. Eigenschaften | 1 | 1 | 2 | 4 | 6 | 8 | 10 | 11 |
|---|---|---|---|---|---|---|---|---|
| Temperatur (°C) | 6500/960 | 680/1020 | 930 | 980 | 830 | 1020 | 850 | 630/990 |
| Temperzeit (h) | 1/3 | 1/2/3/2 | 5 | 3/2 | 50 | 1/2 | 30 | 1/2/3 |
| Biegebruchfestigkeit (MPa) | 90 | 150 | 95 | 100 | 105 | 145 | 125 | 115 |
| Bruchzähigkeit $K_{Ic}$ (MPa m$^{1/2}$) | 1,2 | 1,9 | — | 1,3 | — | 1,7 | 1,5 | — |
| Härte $HV_{0,07}$ | 300 | 420 | — | — | 750 | — | 980 | — |
| Druckfestigkeit (MPa) | 280 | — | 310 | — | 420 | — | 455 | — |
| linearer thermischer Ausdehnungskoeffizient ($K^{-1}$) | $108 \cdot 10^{-7}$ | $115 \cdot 10^{-7}$ | $77 \cdot 10^{-7}$ | $122 \cdot 10^{-7}$ | $93 \cdot 10^{-7}$ | — | $112 \cdot 10^{-7}$ | — |
| maschinelle Bearbeitbarkeit | sehr gut | gut | gut | sehr gut | gut | gut | gut | sehr gut |
| hydrolytische Klasse | 2 | 1–2 | — | 2 | 1 | — | 1 | 2 |
| Farbe | weiß | weiß | weiß | weiß | weiß | weiß | bräunlich | grau/bräunlich |
| Verschleißfestigkeit | gut | — | gut | — | sehr gut | sehr gut | sehr gut | — |
| Rauhtiefe (µm) | 0,25 | — | — | — | 0,15 | — | 0,15 | — |
| Kristallphasenanteil (Vol-%) | | | | | | | | |
| Glimmer | 65 | 65 | 60 | 60 | 45 | 50 | 60 | 70 |
| Cordierit | 5 | 5 | 15 | 10 | 30 | 10 | 15 | 5 |

Die in den Tabellen 1 und 2 ausgeführten Beispiele belegen auch die vorteilhafte erfindungsgemäße Anwendung der Glimmer-Cordierit-Glaskeramik für stomatologische Zwecke.

Zwei weitere ausgewählte Beispiele und zwar die Anwendung der Glimmer-Cordierit-Glaskeramik als Inlay (1) und als Zahnaufbau (2) stellen den nützlichen Effekt der erfindungsgemäßen Anwendung dar:

(1) Zur Herstellung eines Inlays aus Glimmer-Cordierit-Glaskeramik erfolgt zunächst die Präparation der Zahnkavität. Dabei ist auf Anlage von senkrecht aufeinander stehenden Kavitätenwänden mit schwach gerundeten Kanten zu achten. Dünn auslaufende Präparationen sind zu vermeiden. Eine genügend starke Substanzabtragung, welche Schichtstärken von wenigstens 1,0 mm beim Inlay liefert, ist anzustreben. Die Herstellung des zur Einbettung vorgesehenen Modells, welches die Ausmaße und Form der Kavität wiedergibt, kann nach dem direkten Verfahren z. B. durch Wachsmodellation im Munde, oder nach dem indirekten Verfahren, z. B. über eine Abformung und Spezialstumpfherstellung, erfolgen. Nach Fertigstellung des Modells wird ein Gußstift angebracht, der eine Länge von 5 bis 10 mm und einen Durchmesser von 2 - 3 mm besitzt. Das Einbetten erfolgt unter Verwendung von Einbettmassen, wie sie üblicherweise in zahntechnischen Laboratorien zum Guß von hochschmelzenden Dentallegierungen verwendet werden. Vorteilhaft ist die Anwendung eines Vakuum-Virbrationsgerätes, wie es in den zahntechnischen Laboratorien in der Regel vorhanden ist.

Nach Erhärten der Einbettmasse wird der Gußstift entfernt und die Muffel langsam auf eine Temperatur von 500 - 1000°C vorgewärmt. Dabei entweicht der Modellwerkstoff durch den Gußkanal und hinterläßt in der Muffel einen seiner ursprünglichen Form entsprechenden Hohlraum. Das Ausgangsglas wird bei 1450 - 1530°C erschmolzen und bei einer Temperatur von ca. 1250 - 1350°C mittels Schleudergußverfahren oder Vakuumdruckguß in die Muffel gegossen, wobei sich der erwähnte Hohlraum auffüllt. Das Glaskeramikinlay als Ergebnis des Gußvorganges kann nun in der Muffel auf Raumtemperatur abgekühlt werden. Danach wird es durch Ausbetten von der umgebenden Einbettmasse befreit und auf mögliche Fehler untersucht. Ist das Gußobjekt fehlerfrei, wird es erneut eingebettet, wobei eine Einbettmasse gleicher Zusammensetzung wie beim Guß Verwendung findet. Durch Erwärmen mit 10 K/min auf eine Temperatur von 650 - 1050°C über eine Zeit von 0,5 - 24 Stunden erfolgt die Keramisierung in das erfindungsgemäße Glaskeramikinlay. Anschließend wird das Inlay ausgebettet. Eine weitere Möglichkeit besteht in der Überführung des Gußprozesses in den Keramisierungsprozeß, der wiederum bei einer Temperatur von 650 - 1050°C erfolgt, ohne zwischenzeitliches Ausbetten des Gußobjektes. Zur Entfernung von Resten anhaftender Einbettmasse wird das Inlay mit Quarzsand abgestrahlt. Mit einem herkömmlichen Schleifer wird das Inlay vom Gußkanal abgetrennt. Jetzt kann die Einprobe im Mund erfolgen. Die Paßfähigkeit wird kontrolliert z. B. Randschluß, Kontaktpunkte zu den Nachbarzähnen, Okklusion, Korrekturen werden mit Feinkornschleifern vorgenommen. Anschließend erfolgt die Politur auf übliche Weise. Bei bestehender Notwendigkeit, z. B. Einsatz im sichtbaren Bereich, erfolgt eine weitergehende Anpassung an die Farbe des natürlichen Zahnes durch Auftragen von Malfarben und deren Einbrennen oder vorheriges Einbringen von Zusatzkomponenten. Abschließend wird das Inlay mit praxisüblichen Befestigungszementen oder speziellen Haftverfahren in der Kavität verankert.

(2) Zur Herstellung eines Zahnaufbaus aus Glimmer-Cordierit-Glaskeramik in Kombination mit $Al_2O_3$-Sinterkeramik wird zunächst die Abtragung des Zahnes und Ausschachtung des Wurzelkanals vorgenommen. Es erfolgt das Einpassen eines konfektionierten Wurzelstiftes aus $Al_2O_3$-verstärkter Keramik. Der Stift ist z. B. im Querschnitt rund, ist in verschiedenen Stärken entsprechend der Weite des Wurzelkanals verfügbar, z. B.: 1,4 mm, 1,6 mm, 1,8 mm Querschnitt, und verjüngt sich schwach wurzelspitzwärts. Seine Länge muß durch Beschleifen dem Verlauf des Wurzelkanals angepaßt werden. Der eigentliche Aufbau des Zahnes entspricht in seiner Gestalt einem Präparationsstumpf und wird aus Modellwerkstoff nach dem direkten Verfahren modelliert. Die Arbeitsschritte vom Einbetten bis zum Keramisierungsprozeß entsprechen den Darlegungen unter (1).

Zur Überprüfung der Paßfähigkeit wird der Keramik-Zahnaufbau am Patienten einprobiert. Dabei ist auf Randschließverhalten, Länge und korrekte Formgebung zu achten. Die Oberflächenbearbeitung erfolgt mit praxisüblichen Schleif- und Polierkörpern. Das Einzementieren entspricht dem unter (1) dargelegten nach korrekter Wurzelfüllung im apikalen Drittel.

Das Wesen der Erfindung hinsichtlich der Anwendung der Glimmer-Cordierit-Glaskeramik für Verblendschalen soll anhand eines Ausführungsbeispieles näher erläutert werden. In der Zeichnung 1 ist ein vestibular-oraler Querschnitt der erfindungsgemäßen Verblendschale dargestellt.

Die erfindungsgemäße Verblendschale besteht aus einer vestibulären Glaskeramikschicht a, einer Haftvermittlerschicht b und aus einer organischen Kunststoff- oder Verbundstoffschicht c. Die Glaskeramikschicht a ist dabei eine Glimmer-Cordierit-Glaskeramik mit der im Wesen der Erfindung angegebenen Zusammensetzung. Sie wird mittels metallischer Formen gepreßt, keramisiert und oberflächenkonditioniert. Die Oberflächenkonditionierung erfolgt mittels Sandstrahlung mit $Al_2O_3$-Korund in einer Körnung von 50 μm und 2 - 3 bar Druck.

Danach erfolgt eine Reinigung mit Essigsäureethylester im Ultraschallbad. Die labiale bzw. bukkale Fläche der vestibulären Glaskeramikschicht weist die Oberflächen gestalt des jeweils darzustellenden Zahnes auf. Die Incisalkante d ist so gestaltet, daß sie nach lingual verläuft und keine Unterscheidung aufweist. Auf die linguale Fläche der vestibulären Glaskeramikschicht a wird die Haftvermittlerschicht b mittels Silanisierung aufgebracht. Danach wird die organische Kunststoff- oder Verbundstoffschicht c auf die Haftvermittlerschicht b aufgetragen und damit die vestibuläre Glaskeramikschicht a oral hinter-

11

legt. Dabei wird zunächst eine hochbenetzende Kunststoffschicht aufgetragen, die durch eine hochgefüllte, pigmenttragende pastöse Phase eines Verbundstoffes ergänzt wird. Beide Schichten enthalten Fotoinitiatoren und werden gleichzeitig lichtpolymerisiert.

Die erfindungsgemäße Anwendung der Glimmer-Cordierit-Glaskeramik in der Kieferorthopädie/Orthodontie soll anhand von zwei Ausführungsbeispielen näher erläutert werden.

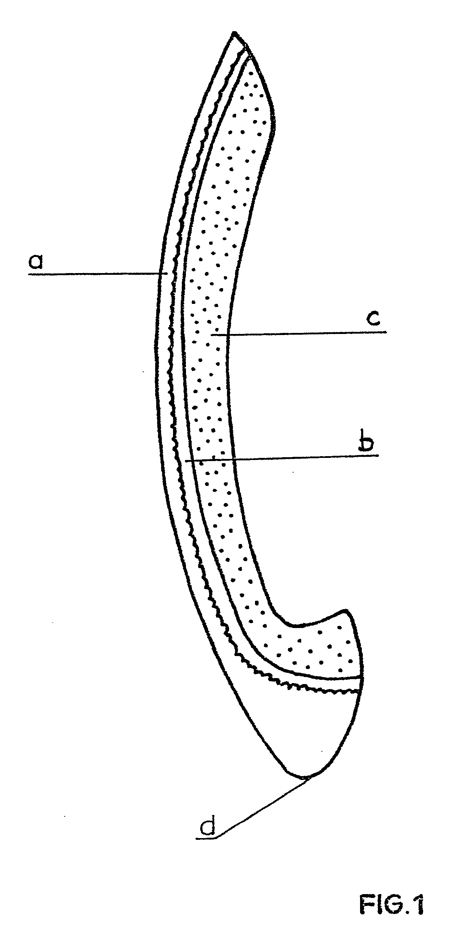

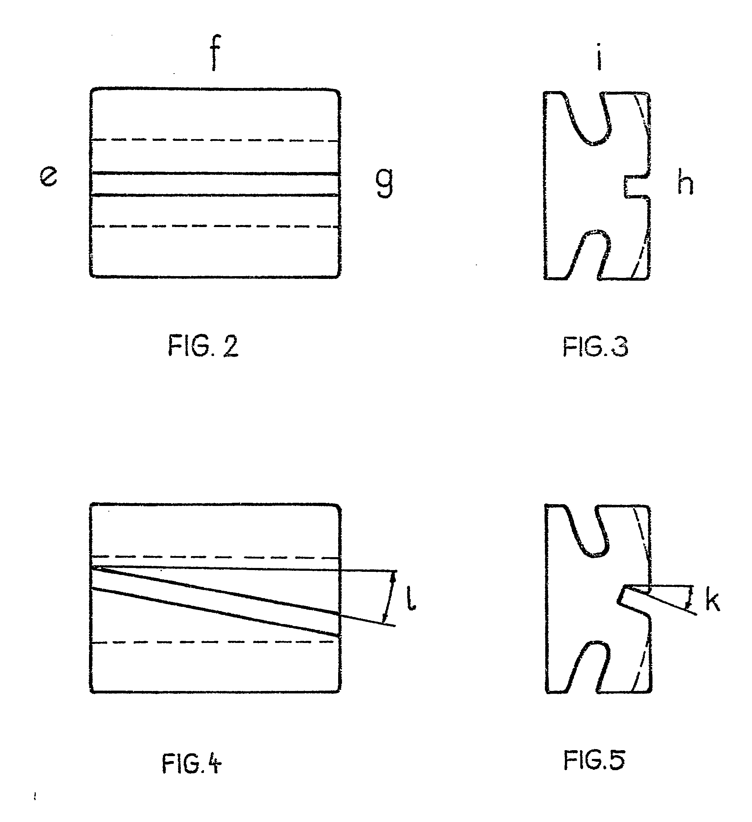

In den Figuren 2; 3; 4 und 4 sind verschiedene Ansichten bzw. Möglichkeiten für die Form von Glaskeramikklebebrackets dargestellt, wobei dio Glaskeramik die im Wesen der Erfindung angegebene Zusammensetzung und Eigenschaften besitzt. Die Basisfläche der Brackets besitzt eine Kantenlänge e von 4,5 mm und f von 2,5 - 6 mm und ist gewölbt oder eben gestaltet und durch Beschleifen mit herkömmlichen Schleifmitteln wird eine mikromechanische Retentionsfläche für orthodontische Adhäsive zur Erzielung von Haftfestigkeiten bis 18 MPa erreicht. Die Höhe i der Brackets ist maximal 3 mm und die Flügel sind entsprechend Figuren 3 und 4 gerade oder abgeschrägt, wobei die Kanten der Glimmer-Cordierit-Glaskeramikklebebrackets gebrochen bzw. abgerundet sind. Der Schlitz der Glaskeramikklebebrackets für die Aufnahme des Regulierungsbogens hat eine Breite g von 0,45 - 0,55 mm und eine Tiefe h von 0,6 - 0,7 mm. Die Angulation des Schlitzes, der Winkel 1 in Figur 4, kann bis 10° betragen und der Torque des Schlitzes, der Winkel k in Figur 5, kann bis 25° groß sein.

Entsprechend den Angaben zur Form, können für die unterschiedlichen Zahntypen standardisierte Grundkörper hergestellt werden.

Nach individueller Anpassung der standardisierten Glaskeramikklebebrackets werden diese, nach Konditionierung des Schmelzes, mittels orthodontischer Kleber, wie z. B. Polymethylmetacrylat, an den Zähnen befestigt und durch Einbinden hochelastischer Regulierungsbögen erfolgt die Korrektur der Zahnstellungsanomalien.

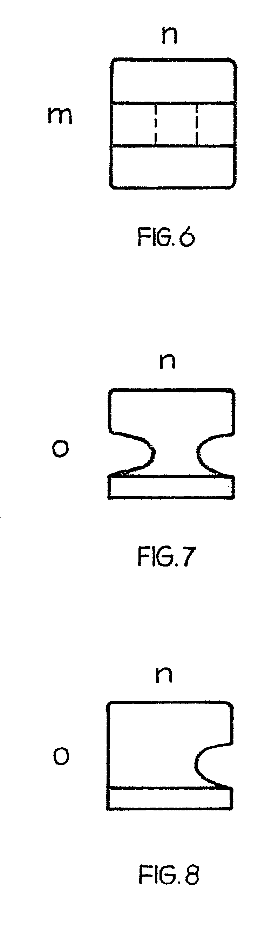

In den Figuren 6; 7 und 8 sind verschiedene Ansichten bzw. Möglichkeiten der Form von Glaskeramikklebeknöpfchen dargestellt, wobei in den Figuren 6 und 7 der Aufbau auf der Basisfläche eine T-Form und in Figur 3 eine Haken-Form besitzt. Die Kanten der Glaskeramikklebeknöpfchen sind ebenfalls gebrochen bzw. abgerundet und die Kanten m und n der Basisfläche sind 2,5 - 3 mm lang und die Höhe o ist maximal 3 mm. Die Glimmer-Cordierit-Glaskeramikklebeknöpfchen werden, wie im ersten Ausführungsbeispiel beschrieben, an den Zähnen befestigt und dienen z. B. zur Aufnahme von Gummizügen.

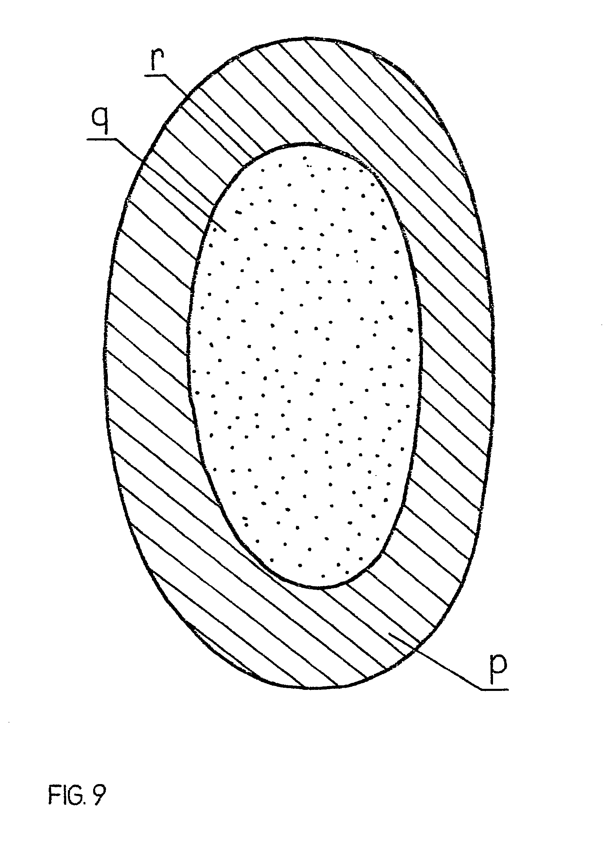

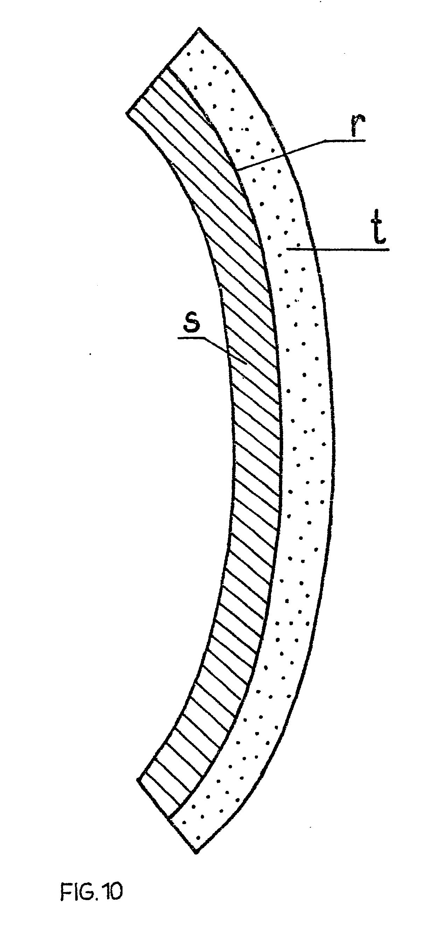

Die erfindungsgemäße Anwendung der Glimmer-Cordierit-Glaskeramik für Implantatmaterial in der Kopf-Hals-Chirurgie wird in zwei Ausführungsbeispielen anhand von 2 Figuren näher erläutert (Figuren 9 und 10). Es zeigen:

Figur 9 - einen Implantatkörper für die Nasennebenhöhlenchirurgie mit einer bioaktiven Randzone,

Figur 10 - einen Implantatkörper für die Nasennebenhöhlenchirurgie mit einer bioaktiven Oberflächenschicht. Der in Figur 9 dargestellte Implantatkörper für die Nasennebenhöhlenchirurgie besteht aus einer bioaktiven Randzone p und einer biokompatiblen Glimmer-Cordierit-Glaskeramikkernzone q. Die Randzone p und die Kernzone q sind an ihrer Phasengrenze r durch Einbringen von Farboxiden, wie z. B. $FeO/Fe_2O_3, MnO_2, Cr_2O_3$ oder $NiO$ sichtbar markiert. Die Oberflächengestalt kann eben oder entsprechend den anatomischen Bedingungen gekrümmt sein.

Die Ausführung der Randzone p aus hochbioaktiver Glaskeramik garantiert einen festen Knochenverbund. Die Kernzone q begünstigt die Epithelisierung aufgrund der Eigenschaften der verwendeten biokompatiblen Glimmer-Cordierit-Glaskeramik.

Das 2. Ausführungsbeispiel wird mit Figur 10 dargestellt, wobei die eine Oberflächenschicht s aus der hochbioaktiven Glaskeramik und die andere Oberflächenschicht t aus der biokompatiblen Glimmer-Cordierit-Glaskeramik besteht. Beide Oberflächenschichten (s und t) sind an ihrer Phasengrenze, wie bereits im 1. Ausführungsbeispiel beschrieben, verbunden.

**Patentansprüche**

1. Glimmer-Cordierit-Glaskeramik mit guter chemischer Beständigkeit und ausgezeichneter maschineller Bearbeitbarkeit sowie einer hohen Verschleissfestigkeit, dadurch gekennzeichnet, dass sie eine Zusammensetzung in Masse-% von

| | | | |
|---|---|---|---|
| $SiO_2$ | 43 | – | 50 |
| $Al_2O_3$ | 26 | – | 30 |
| $MgO$ | 11 | – | 15 |
| $R_2O$ | 7 | – | 10,5 |
| $F^-$ | 3,3 | – | 4,8 |
| $Cl^-$ | 0,01 | – | 0,6 |
| $CaO$ | 0,1 | – | 3 |
| $P_2O_5$ | 0,1 | – | 5 |
| $BaO, SrO, PbO$ | 0 | – | 8 |
| Farbkomponenten | 0 | – | 4 |

besitzt, wobei $R_2O$ die Summe aus 3 - 5,5 Masse-% $Na_2O$ und 4 - 6 Masse-% $K_2O$ darstellt, und als Kristallphasen Glimmer neben 5 - 30 Vol.-% Cordierit enthält, wobei das Gefüge dadurch charakterisiert ist, dass relativ grosse Glimmerkristalle von 10 - 200 μm Grösse in die Glasmatrix eingebettet vorliegen und Cordieritkristalle von 0,5 - 5 μm Grösse angeordnet sind, wobei auch kleine, ebene Glimmer von 0,5 - 5 μm Grösse auftreten können.

2. Glimmer-Cordierit-Glaskeramik nach Anspruch 1, mit einer Bruchzähigkeit als $K_{IC}$-Wert bis 2,0 MPa $m^{1/2}$, einer Härte als $HV_{0,07}$ von 300 - 1000, einer Druckfestigkeit bis 450 N/mm², und einem linearen thermischen Ausdehnungskoeffizienten von 75 - 125 x $10^{-7}$ $K^{-1}$, dadurch gekennzeichnet, dass sie die Komponenten BaO, SrO und PbO einzeln oder im Gemisch bis 8 Masse-% enthält.

3. Glimmer-Cordierit-Glaskeramik nach Anspruch 1 oder 2, dadurch gekennzeichnet, daß sie Farbkomponenten, wie z. B. NiO, $Cr_2O_3$, $MnO_2$, FeO, $Fe_2O_3$, $TiO_2$, einzeln oder im Gemisch bis 4 Masse-% enthält.

4. Glimmer-Cordierit-Glaskeramik nach Anspruch 1 bis 3 für stomatologische Zwecke, dadurch gekennzeichnet, daß sie für Inlays, Kronen, Aufbauten und Brücken, vorzugsweise für das Seitenzahngebiet, anwendbar ist, die äußere Form je nach anatomischer Besonderheit individuell aus der reinen Glimmer-Cordierit-Glaskeramik gestaltbar ist oder die Glimmer-Cordierit-Glaskeramik auf einen hochfesten Grundkörper, z. B. $Al_2O_3$-Sinterkeramik oder Metalle, aufbringbar ist, z. B. durch "Angießen", wodurch der Grundkörper vollständig oder teilweise umhüllt ist.

5. Glimmer-Cordierit-Glaskeramik nach Anspruch 1 bis 3 für stomatologische Zwecke, dadurch gekennzeichnet, daß die Dentalprodukte, wie z. B. Inlays, Kronen und Brücken, die aus der reinen Glimmer-Cordierit-Glaskeramik bestehen, einen einstellbaren linearen thermischen Ausdehnungskoeffizienten von 80 - 120 x $10^{-7}$ $K^{-1}$, eine Biegebruchfestigkeit von mehr als 80 MPa, eine Druckfestigkeit bis 450 N/mm², eine einstellbare Härte von 300 - 800 $HV_{0,07}$, eine sehr gute chemische Beständigkeit, z. B. eine hydrolytische Beständigkeit der Klasse 1 oder 2 aufweisen, daß die Dentalprodukte eine Rauhtiefe von 0,15 μm und eine Röntgenopazität aufweisen und gleichzeitig maschinell bearbeitbar sind, wobei diese Parameter auf die Eigenschaften des natürlichen Zahnschmelzes eingestellt sind, und daß bei Verbundwerkstoffen aus Glimmer-Cordierit-Glaskeramik und hochfesten Grundkörpern, wie z. B. Metallen oder Sinterkorund, zusätzlich derern Bruchfestigkeit auf 200 - 400 MPa gesteigert ist.

6. Glimmer-Cordierit-Glaskeramik nach Anspruch 1 bis 5 für stomatologische Zwecke, dadurch gekennzeichnet, daß sie mit herkömmlichen Befestigungszementen oder nach Silanisierung mit Kompositwerkstoffen mit der Zahnhartsubstanz verbindbar ist.

7. Glimmer-Cordierit-Glaskeramik nach Anspruch 1 bis 3 für Verblendschalen, zur Verblendung von Kunststoffzähnen, Metallgerüsten oder natürlichen Zähnen, dadurch gekennzeichnet, daß die Verblendschalen vestibulär aus einer Glimmer-Cordierit-Glaskeramikschicht und oral aus einer organischen Kunststoff- oder Verbundstoffschicht bestehen, wobei beide Schichten mittels einer Haftvermittlerschicht verbunden sind.

8. Glimmer-Cordierit-Glaskeramik nach Anspruch 1 bis 3 und 7 für Verblendschalen, dadurch gekennzeichnet, daß die Stärke der vestibulären Glaskeramikschicht gleich oder kleiner 0,5 mm und im Bereich der Incisalkante größer 0,5 mm ist.

9. Glimmer-Cordierit-Glaskeramik nach Anspruch 7 und 8 für Verblendschalen, dadurch gekennzeichnet, daß die orale Seite der vestibulären Glaskeramikschicht oberflächenvergrößernde Strukturen aufweist.

10. Glimmer-Cordierit-Glaskeramik nach Anspruch 1 bis 3 für Anwendungen in der Kieferorthopädie/Orthodontie, dadurch gekennzeichnet, daß sie für Klebebrackets und Klebeknöpfchen einsetzbar ist, wobei die konfektionierte Form dieser Teile eine Mindestwandstärke von 1 mm und eine Höhe bis maximal 3 mm besitzt, die Bodenfläche gewölbt oder eben ist und zur Erzielung einer hohen Haftfestigkeit vorzugsweise eine große Rauhigkeit von mehr als 5 μm aufweist, die Breite der Bodenfläche 2,5 - 6,0 mm beträgt, die Flügel gerade oder abge schrägte Kanten und die Klebebrackets einen Schlitz von 0,45 - 0,55 mm Breite und einer Tiefe von 0,63 - 0,71 mm besitzen, wobei aufgrund der ausgezeichnetten Bearbeitbarkeit der Glimmer-Cordierit-Glaskeramik eine der Anatomie des Zahnes und der mechanischen Beanspruchung der Teile, z. B. der Klebebrackets, angepaßte individuelle Formgebung selbst während des klinischen Einsatzes gesichert ist, z. B. Winkligkeit erzielbar ist bzw. die Basisfläche in ihrer Wölbung exakt anpaßbar ist, wodurch bei Anwendung von Klebemitteln, wie z. B. orthodontischen Klebern, insbesondere Polymethylmetacrylat oder anorganischorganischen Verbundwerkstoffen, die hohe Retentionskapazität der Glimmer-Cordierit-Glaskeramik zu einer hohen Verbundfestigkeit, z. B. 18 MPa, führt und wobei aufgrund des zahnfarbenen Aussehens der Glimmer-Cordierit-Glaskeramik eine günstige ästhetische Wirkung erzielt wird.

11. Glimmer-Cordierit-Glaskeramik nach Anspruch 10 für Anwendungen in der Kieferorthopädie/Orthodontie, dadurch gekennzeichnet, daß der Schlitz der Klebebrackets eine Schrägstellung zur Basisfläche, einen Torque bis 25° und eine Angulation bis 10° aufweist, der Schlitz aus Metall sein kann und vorzugsweise in das Glaskeramikklebebracket eingeklebt ist.

12. Glimmer-Cordierit-Glaskeramik nach Anspruch 10 für Anwendungen in der Kieferorthopädie/Orthodontie, dadurch gekennzeichnet, daß die aus der Glimmer-Cordierit-Glaskeramik bestehenden Klebeknöpfchen eine Form mit einer Basisfläche von 2,5 bis 3 mm Kantenlänge besitzen und der Aufbau auf der Basisfläche entweder eine T- oder Haken-Form besitzt.

13. Glimmer-Cordierit-Glaskeramik nach Anspruch 1 bis 3 als Implantationsmaterial für die Kopf-Hals-Chirurgie, dadurch gekennzeichnet, daß das Implantationsmaterial entweder nur aus der Glimmer-Cordierit-Glaskeramik oder aus einer Kombination einer maschinell boarbeitbaren, hochbioaktiven Glaskeramik mit der biokompatiblen Glimmer-Cordierit-Glaskeramik besteht.

14. Glimmer-Cordierit-Glaskeramik nach Anspruch 13 als Implantationsmaterial für die Kopf-Hals-Chirurgie, dadurch gekennzeichnet, daß die Kombination der maschinell bearbeitbaren hochbioaktiven Glaskeramik mit der biokompatiblen Glimmer-Cordierit-Glaskeramik in der Erweichungsphase aufgrund entsprechend abgestimmter linearer thermischer Ausdehnungskoeffizienten herstellbar ist.

15. Glimmer-Cordierit-Glaskeramik nach Anspruch 13 und 14 als Implantationsmaterial für die Kopf-Hals-Chirurgie, dadaurch gekennzeichnet, daß die Phasengrenze zwischen beiden zu verbindenden Glaskeramiken durch Einbringen geringer Farboxidmengen vor der thermischen Behandlung der Ausgangsprodukte als farbige Grenzschicht sichtbar ist.

16. Glimmer-Cordierit-Glaskeramik nach Anspruch 1 bis 3 und 13 als Implantationsmaterial für die Kopf-Hals-Chirurgie, dadurch gekennzeichnet, daß sie eine geringe Löslichkeit und eine hohe Oberflächenladung aufgrund des speziellen Glimmer-Cordierit-Gefüges und der resultierenden Restglasphase aufweist.

17. Glimmer-Cordierit-Glaskeramik nach Anspruch 1 bis 3 und 13 bis 16 als Implantationsmaterial für die Kopf-Hals-Chirurgie, dadurch gekennzeichnet, daß sie intraoperativ mit konventionellen Operationsinstrumenten bearbeitbar ist.

## Claims

1. Mica-cordierite glass-ceramic having good chemical resistance and excellent machinability as well as high resistance to wear, characterized in that it has a composition in percent by mass of

| | | | |
|---|---|---|---|
| $SiO_2$ | 43 | – | 50 |
| $Al_2O_3$ | 26 | – | 30 |
| MgO | 11 | – | 15 |
| $R_2O$ | 7 | – | 10.5 |
| $F^-$ | 3.3 | – | 4.8 |
| $Cl^-$ | 0.01 | – | 0.6 |
| CaO | 0.1 | – | 3 |
| $P_2O_5$ | 0.1 | – | 5 |
| BaO, SrO, PbO | 0 | – | 8 |
| Colour components | 0 | – | 4 |

$R_2O$ representing the sum of 3–5.5% by mass of $Na_2O$ and 4–6% by mass of $K_2O$, and contains as crystal phases mica in addition to 5–30% by volume of cordierite, the structure being characterized in that there are relatively large mica crystals of from 10–200 μm in size embedded in the glass matrix, and cordierite crystals of from 0.5–5 μm in size are disposed, it also being possible for small, flat micas of from 0.5–5 μm in size to occur.

2. Mica-cordierite glass-ceramic according to claim 1, having a breaking ductility as $K_{IC}$ value of up to 2.0 MPa $m^{1/2}$, a hardness as $HV_{0.07}$ of 300–1000, a compression strength of up to 450 N/mm², and a linear coefficient of thermal expansion of from $75–125 \times 10^{-7}$ $K^{-1}$, characterized in that it contains the components BaO, SrO and PbO singly or as a mixture up to 8% by mass.

3. Mica-cordierite glass-ceramic according to claim 1 or 2, characterized in that that it contains colour components such as e.g. NiO, $Cr_2O_3$, $MnO_2$, FeO, $Fe_2O_3$, $TiO_2$, singly or as a mixture up to 4% by mass.

4. Mica-cordierite glass-ceramic according to claims 1 to 3 for stomatological purposes, characterized in that it is usable for inlays, crowns, constructs, and bridges, preferably for the lateral dental area, the outer shape is individually formable of the pure mica-cordierite glass-ceramic according to anatomical particularity, or the mica-cordierite glass-ceramic can be applied to a high-strength foundation, e.g. $Al_2O_3$ sintered ceramic or metals, e.g. by "casting-on", whereby the foundation is completely or partially enveloped.

5. Mica-cordierite glass-ceramic according to claims 1 to 3 for stomatological purposes, characterized in that the dental products, such as e.g. inlays, crown, and bridges made of the pure mica-cordierite glass-ceramic have an adjustable linear coefficient of thermal expansion of from $80–120 \times 10^{-7}$ $K^{-1}$, a bending strength of more than 80 MPa, a compression strength of up to 450 N/mm², an adjustable hardness of from 300–800 $HV_{0.07}$, very good chemical resistance, e.g. a hydrolytic resistance of Class 1 or 2, that the dental products have a peak-to-valley height of 0.15 μm and radiopacity and are at the same time machinable, these parameters being adjusted to the properties of the natural tooth enamel, and that in the case of composite materials made of mica-cordierite glass-ceramic and high-strength base bodies, such as e.g. metals or sintered corundum, the breaking strength thereof is additionally increased to from 200–400 MPa.

6. Mica-cordierite glass-ceramic according to claims 1 to 5 for stomatological purposes, characterized in that it is joinable to the tooth enamel by means of conventional attaching cements or after silanization with composite materials.

7. Mica-cordierite glass-ceramic according to claims 1 to 3 for veneer shells, for the veneering of plastics teeth, metal trestles or natural teeth, characterized in that the veneer shells are made vestibularly of a mica-cordierite glass-ceramic layer and orally of an organic plastics or composite material layer, the two layers being joined by means of a layer of an adhesive agent.

8. Mica-cordierite glass-ceramic according to claims 1 to 3 and 7 for veneer shells, characterized in that the thickness of the vestibular glass-ceramic layer is equal to or less than 0.5 mm and in the region of the incisal edge greater than 0.5 mm.

9. Mica-cordierite glass-ceramic according to claims 7 and 8 for veneer shells, characterized in that the oral side of the vestibular glass-ceramic layer has surface-enlarging structures.

10. Mica-cordierite glass-ceramic according to claims 1 to 3 for applications in orthodontics, characterized in that it can be utilized for adhesive brackets and adhesive buttons, the ready-made form of these parts having a minimum wall thickness of 1 mm and a height of up to 3 mm maximum, the bottom surface is curved or flat and preferably has a high roughness of more than 5 μm for achieving high adhesive strength, the width of the bottom surface is from 2.5–6.0 mm, the wings have straight or chamfered edges, and the adhesive brackets have a slot from 0.45–0.55 mm wide and from 0.63–0.71 mm deep, an individual fashioning adapted to the anatomy of the tooth and to the mechanical stress on the parts, e.g. the adhesive brackets, being ensured even during clinical use owing to the excellent machinability of the mica-cordierite glass-ceramic, e.g. angularity being achievable and the base surface being exactly adaptable in its curvature, whereby upon application of adhesives, such as e.g. orthodontic adhesives, particularly polymethylmethacrylate or inorganic-organic composite materials, the high retention capacity of the mica-cordierite glass-ceramic leads to high adhesive strength, e.g. 18 MPa, and a favourable aesthetic effect being achieved owing to the tooth-coloured appearance of the mica-cordierite glass-ceramic.

11. Mica-cordierite glass-ceramic according to claim 10 for applications in orthodontics, characterized in that the slot of the adhesive brackets has an obliquity to the base surface, a torque of up to 25° and an angulation of up to 10°, the slot may be of metal and is preferably cemented into the glass-ceramic adhesive bracket.

12. Mica-cordierite glass-ceramic according to claim 10 for applications in orthodontics, characterized in that the adhesive buttons made of the mica-cordierite glass-ceramic have a shape with a base surface of from 2.5 to 3 mm edge length, and the construct on the base surface has either a T-shape or a hook-shape.

13. Mica-cordierite glass-ceramic according to claims 1 to 3 as implantation material for head-neck surgery, characterized in that the implantation material is made either only of the mica-cordierite glass-ceramic or of a combination of a machinable, highly bioactive glass-ceramic with the biocompatible mica-cordierite glass-ceramic.

14. Mica-cordierite glass-ceramic according to claim 13 as implantation material for head-neck surgery, characterized in that the combination of the machinable, highly bioactive glass-ceramic with the biocompatible mica-cordierite glass-ceramic is producible in the softening phase owing to appropriately harmonized linear coefficients of thermal expansion.

15. Mica-cordierite glass-ceramic according to claims 13 and 14 as implantation material for head-neck surgery, characterized in that the phase boundary between the two glass-ceramics to be combined is visible as a coloured boundary layer through introduction of small amounts of colouring oxides prior to the thermal treatment of the starting products.

16. Mica-cordierite glass-ceramic according to claims 1 to 3 and 13 as implantation material for head-neck surgery, characterized in that it has low solubility and a high surface charge owing to the special mica-cordierite structure and the resultant residual glass phase.

17. Mica-cordierite glass-ceramic according to claims 1 to 3 and 13 to 16 as implantation material for head-neck surgery, characterized in that it is workable with conventional operating instruments during the operation.

**Revendications**

1. Vitrocéramique de cordiérite et de mica avec une bonne résistance chimique et une excellente usinabilité ainsi qu'une grande résistance à l'usure, caractérisé en ce qu'elle possède une composition en %-masse de

| | | | |
|---|---|---|---|
| $SiO_2$ | 43 | – | 50 |
| $Al_2O_3$ | 26 | – | 30 |
| $MgO$ | 11 | – | 15 |
| $R_2O$ | 7 | – | 10,5 |
| $F^-$ | 3,3 | – | 4,8 |
| $Cl^-$ | 0,01 | – | 0,6 |
| $CaO$ | 0,1 | – | 3 |
| $P_2O_5$ | 0,1 | – | 5 |
| $BaO, SrO, PbO$ | 0 | – | 8 |
| Colorants | 0 | – | 4 |

$R_2O$ représentant la somme de 3–5,5%-masse de $Na_2O$ et de 4–6%-masse de $K_2O$, cette vitrocéramique contenant, outre 5–30% en volume de cordiérite, du mica comme phase cristalline, la structure étant caractérisée par de relativement grands cristaux de mica, de 10–200 µm, encastrés dans la matrice vitreuse, et des cristaux de cordiérite de 0,5–5 µm, de petits plans de mica de 0,5–5 µm pouvant également se présenter.

2. Vitrocéramique de cordiérite et de mica selon la revendication 1, avec une ténacité à la rupture en valeur $K_{IC}$ jusqu'à 2,0 MPa $m^{1/2}$, une dureté en valeur $HV_{0,07}$ de 300–1000, une résistance à la compression allant jusqu'à 450 $N/mm^2$, et un coefficient d'allongement thermique linéaire de 75–125 × $10^{-7}K^{-1}$, caractérisée en ce qu'elle comprend les composants BaO, SrO et PbO, séparément ou en mélange, jusqu'à 8% en masse.

3. Vitrocéramique de cordiérite et de mica selon les revendications 1 ou 2, caractérisée en ce qu'elle comprend les colorants, comme par exemple NiO, $Cr_2O_3$, $MnO_2$, FeO, $Fe_2O_3$, $TiO_2$, séparément ou en mélange, jusqu'à 4% en masse.

4. Vitrocéramique de cordiérite et de mica selon les revendications 1 à 3, pour un usage stomatologique, caractérisée en ce qu'elle est utilisable pour des implants, des couronnes, des reconstructions et des ponts, de préférence pour les dents latérales, la forme extérieure de chacune étant façonnable selon les particularités anatomiques individuelles à partir d'une vitrocéramique de cordiérite et de mica pure, ou la vitrocéramique de cordiérite et de mica pouvant être déposée sur un corps de base de haute résistance, par ex. $Al_2O_3$-céramique amalgame ou métal, p.ex. par "moulage", par quoi le corps de base est recouvert entièrement ou en partie.

5. Vitrocéramique de cordiérite et de mica selon les revendications 1 à 3, pour un usage stomatologique, caractérisée en ce que les produits dentaires, comme p.ex. implants, couronnes et ponts composés de vitrocéramique de cordiérite et de mica pure, présentent un coefficient d'allongement thermique linéaire ajustable de 80–120 × $10^{-7}$ $K^{-1}$, une résistance à la flexion de plus de 80 MPa, une résistance à la compression jusqu'à 450 $N/mm^2$, une dureté ajustable de 300–800 $HV_{0,07}$, une très bonne résistance chimique, p.ex. présentant une résistance hydrolytique de classe 1 ou 2, et en ce que les produits dentaires présentent une profondeur de rugosité de 0,15 µm et une opacité aux rayons X tout en étant usinables, avec ces paramètres étant ajustables par rapport aux caractéristiques des dents naturelles, et en ce que, par réunion des matières vitrocéramiques de cordiérite et de mica avec des corps de base de haute résistance, comme p.ex. des métaux ou amalgames, la résistance à la rupture est encore augmentée à 200–400 MPa.

6. Vitrocéramique de cordiérite et de mica selon les revendications 1 à 5, pour un usage stomatologique, caractérisée en ce qu'elle est liable avec la substance dure de la dent avec des ciments de consolidation traditionnels ou après utilisation de silane avec des matériaux composites.

7. Vitrocéramique de cordiérite et de mica selon les revendications 1 à 3, pour enveloppes de revêtement, pour revêtement de dents synthétiques à carasse métallique ou dents naturelles, caractérisée en ce que les enveloppes de revêtement se composent d'une couche vestibulaire de vitrocéramique de cordiérite et de mica et d'une couche orale de matière synthétique organique ou de liaison, les deux couches étant liées au moyen d'une couche assurant l'adhésion.

8. Vitrocéramique de cordiérite et de mica selon les revendications 1 à 3 et 7, pour enveloppes de revêtement, caractérisée en ce que l'épaisseur de la couche vestibulaire de vitrocéramique est égale ou inférieure à 0,5 mm et est plus épaisse que 0,5 mm dans la zone d'incision.

9. Vitrocéramique de cordiérite et de mica selon les revendications 7 et 8, pour enveloppes de revêtement, caractérisée en ce que la face orale de la couche vestibulaire de vitrocéramique présente une structure superficielle agrandissant la surface.

10. Vitrocéramique de cordiérite et de mica selon les revendications 1 à 3, pour utilisation en orthopédie des mâchoires/orthodontie, caractérisée en ce qu'elle est utilisable pour des crochets ou des petits boutons de collage, la forme façonnée de ces parties ayant une épaisseur de paroi d'au minimum 1 mm et une hauteur maximum de 3 mm, la surface de base étant cintrée ou plane, et présentant, de manière préférentielle, une grande rugosité de plus de 5 µm, pour obtenir une plus grande solidité de fixation, la largeur de la surface de base ayant 2,5–6 mm, les ailes présentant des bords droits ou obliques et les crochets de collage ayant une entaille de 0,45–0,55 mm de large et de 0,63–0,71 mm de profondeur, étant avéré que, en vertu de l'excellente usinabilité de la vitrocéramique de cordiérite et de mica, un façonnage individuel s'appliquant à l'anatomie des dents et au comportement mécanique des parties, p.ex. des crochets de collage, se trouve assuré, même pendant l'intervention clinique, l'angularité pouvant p.ex. être obtenue, ou encore la surface de base pouvant être ajustée exactement selon sa courbure, par quoi, par l'emploi de moyens de collage, comme p.ex. des colles orthodontiques, en particulier des polyméthacrylates ou des matériaux de collage inorganiques ou organiques, la grande capacité de rétention de vitrocéramique de cordiérite et de mica mène à une grande résistance de collage, p.ex. 10 MPa, l'aspect de couleur de dent de la vitrocéramique de cordiérite et de mica permettant l'obtention d'un effet esthétique favorable.

11. Vitrocéramique de cordiérite et de mica selon la revendication 10, pour utilisation en orthopédie des mâchoires/orthodontie, caractérisée en ce que l'entaille des crochets de collage présente une position oblique par rapport à la surface de base, selon une torsion jusqu'à 25° et un angle jusqu'à 10°, l'entaille pouvant être en métal, et étant de préférence collée dans le crochet de collage en vitrocéramique.

12. Vitrocéramique de cordiérite et de mica selon la revendication 10, pour utilisation en orthopédie des mâchoires/orthodontie, caractérisée en ce que les petits boutons de collage en vitrocéramique de cordiérite et de mica ont une forme avec une surface de base ayant une longueur de flanc de 2,5–3 mm, la structure construite sur la surface de base ayant soit une forme de T, soit une forme en crochet.

13. Vitrocéramique de cordiérite et de mica selon les revendications 1 à 3, pour utilisation comme matériel d'implantation pour la chirurgie de la tête et du cou, caractérisée en ce que le matériel d'implantation se compose soit seulement de vitrocéramique de cordiérite et de mica, soit d'une combinaison d'une vitrocéramique usinable, à haute bioactivité, avec la vitrocéramique de cordiérite et de mica biocompatible.

14. Vitrocéramique de cordiérite et de mica selon la revendication 13, pour utilisation comme matériel d'implantation pour la chirurgie de la tête et du cou, caractérisée en ce que la combinaison de la vitrocéramique usinable, à haute bioactivité avec la vitrocéramique de cordiérite et de mica biocompatible est fabricable dans la phase de ramollissement, en vertu des coefficients d'allongement thermique linéaire conformes et compatibles.

15. Vitrocéramique de cordiérite et de mica selon les revendications 13 et 14, pour utilisation comme matériel d'implantation pour la chirurgie de la tête et du cou, caractérisée en ce que la limite des phases entre les deux vitrocéramiques à assembler est visible, par l'adjonction d'une petite quantité d'oxydes colorants comme couche limite colorée, avant le traitement thermique du produit fini.

16. Vitrocéramique de cordiérite et de mica selon les revendications 1 à 3 et 13, pour utilisation comme matériel d'implantation pour la chirurgie de la tête et du cou, caractérisée en ce qu'elle présente une faible solubilité et une charge superficielle élevée en vertu des textures spéciales de la cordiérite de mica et de la phase vitrocéramique restante qui en résulte.

17. Vitrocéramique de cordiérite et de mica selon les revendications 1 à 3 et 13 à 16, pour utilisation comme matériel d'implantation pour la chirurgie de la tête et du cou, caractérisée en ce qu'elle est usinable intraopérativement avec des instruments d'opération conventionnels.

FIG.1

FIG. 2

FIG.3

FIG.4

FIG.5

n

m

FIG. 6

n

o

FIG. 7

n

o

FIG. 8

FIG. 9

r

t

s

FIG.10